# EUROPEAN PATENT APPLICATION

(11) **EP 3 106 076 A1**
(43) Date of publication of application: **21.12.2016**
(21) Application number: 14881714.1
(22) Date of filing: 29.09.2014
(51) Int. Cl.: A61B 1/00

(54) **RETAINING MECHANISM FOR ENDOSCOPE GUIDE MEMBER, AND ENDOSCOPE**

(30) Priority: 10.02.2014 JP 2014023639
(71) Applicant: OLYMPUS CORPORATION, Hachioji-shi Tokyo 192-8507 (JP)
(72) Inventor: SUEYASU, Hidetada, Tokyo 192-8507 (JP); MATSUURA, Wataru, Tokyo 192-8507 (JP); KUDO, Ryota, Tokyo 192-8507 (JP); OUCHI, Naoya, Tokyo 192-8507 (JP)
(74) Representative: Gunzelmann, Rainer
(86) International application number: PCT/JP2014/075910
(87) International publication number: WO 2015/118719

(57) **Abstract**

A holding mechanism 500 has holding portions 501,503. The holding portion 501 holds a part 301a which is exposed to the outside of an endoscope 10. The holding portion 503 is disposed at a position different from that of the holding portion 501. The holding portion 503 holds another part 301b which is exposed to the outside of the endoscope 10, in an orientation different from an orientation of the holding portion 501.

## Description

### Technical Field

The present invention relates to a holding mechanism for an endoscopic guide member which holds the guide member to guide, to a subject, a treatment instrument projecting from an opening portion disposed in an inserting section of an endoscope, and an endoscope having this holding mechanism.

### Background Art

In general, when a treatment instrument treats a subject such as a bile duct, there are used a side view type of endoscope, and a guide member that guides the treatment instrument to the subject.

In this case, the treatment instrument has a cylinder member which is disposed in a distal end portion of the treatment instrument and into which the guide member can be inserted. The cylinder member is slidable along the guide member so that the guide member is inserted into the cylinder member. By this sliding, the cylinder member functions as a monorail portion, and the treatment instrument is movable along the guide member.

The guide member is formed by, e.g., a fine linear member.

The guide member is inserted to a treatment instrument inserting channel that communicates with a treatment instrument insertion port portion from a forceps plug portion disposed in a grasping portion of the endoscope via the treatment instrument insertion port portion. Further, a distal end portion of the guide member is inserted into the treatment instrument inserting channel, and projects from a distal end opening portion disposed in a distal end portion of an inserting section of the endoscope. In the side view type of endoscope, the distal end opening portion is disposed in a side surface of the distal end portion. Consequently, the distal end portion of the guide member projects to a lateral side of the distal end portion of the inserting section. Further, the distal end portion of the guide member reaches the subject. It is to be noted that a proximal end portion of the guide member is exposed from the treatment instrument insertion port portion to the outside of the endoscope.

The treatment instrument moves along the guide member by the cylinder member so that the guide member passes through the cylinder member. At this time, the treatment instrument is guided by the guide member to be inserted to the treatment instrument inserting channel from the forceps plug portion via the treatment instrument insertion port portion. Further, the treatment instrument is guided by the guide member to be inserted into the treatment instrument inserting channel, and projects from the distal end opening portion to the lateral to reach the subject.

In this way, the treatment instrument is guided to the subject by the guide member.

Such guide members are disclosed in, e.g., Patent Literature 1 and Patent Literature 2.

In Patent Literature 1 and Patent Literature 2, a proximal end portion of a guide member is held by a holding member separate from an endoscope so that the guide member is fixed to prevent positional shift of the guide member. The holding member is attached to a treatment instrument insertion port portion, to be attachable to and detachable from the endoscope.

### Citation List

### Patent Literatures

Patent Literature 1: Jpn. PCT National Publication No. 2008-529723
Patent Literature 2: Jpn. Pat. Appln. KOKAI Publication No. 2003-116777

### Summary of Invention

### Technical Problem

In Patent Literature 1 and Patent Literature 2, a holding member is separate from an endoscope, and hence, a great deal of time and labor are required to attach the holding member to the endoscope. The holding member is separate from the endoscope, and hence, a high cost is required. When a treatment instrument moves forward and backward along an axial direction of the treatment instrument, a guide member might shift due to the forward and backward movement.

Thus, it is demanded that the time and labor for the attaching be suppressed, the holding member be inexpensive, and the guide member be prevented from shifting.

The present invention has been made in view of these circumstances, and objects thereof are to provide a holding mechanism for an endoscopic guide member which capable of suppressing time and labor for attaching, being inexpensive, and preventing the guide member from shifting, and to provide an endoscope having this holding mechanism.

### Solution to Problem

An aspect of a holding mechanism for an endoscopic guide member of the present invention is a holding mechanism for the endoscopic guide member which holds the guide member that is inserted to a lumen through an inserting section of an endoscope to be inserted to the lumen and that projects from an opening portion disposed in the inserting section to guide a treatment instrument, the holding mechanism includes a first holding portion which is disposed in an operating section that operates the endoscope and that is coupled to a proximal end portion of the inserting section, the first holding portion holding, in the operating section, a part of the guide member which is exposed from the endoscope on a proximal end side of the guide member in a state where a distal end portion of the guide member projects from the opening portion; and a second holding portion which is disposed at a position different from that of the first holding portion in the operating section, the second holding portion holding the guide member at a position different from that of the part held by the first holding portion on the proximal end portion side of the guide member and in an orientation different from an orientation of the first holding portion.

### Brief Description of Drawings

FIG. 1A is a schematic perspective view of a side view type endoscope according to a first embodiment of the present invention;
FIG. 1B is a top plan view of a distal end hard portion;
FIG. 2A is a schematic view of a holding mechanism for an endoscopic guide member in the first embodiment;
FIG. 2B is a vertical cross-sectional view showing one example of a configuration of a groove portion;
FIG. 2C is a transverse cross-sectional view showing one example of the configuration of the groove portion;
FIG. 3A is a view to explain that a contrasting tube is inserted to a bile duct;
FIG. 3B is a view to explain that the guide member is inserted to the bile duct;
FIG. 3C is a view to explain that a treatment instrument is guided by the guide member to be inserted to the bile duct;
FIG. 3D is a perspective view of a cylinder member into which the guide member is inserted;
FIG. 4A is a schematic view of a holding mechanism in Modification 1 of the first embodiment;
FIG. 4B is a top plan view of a holding portion including a forceps plug portion shown in FIG. 4A;
FIG. 5A is a schematic perspective view of a holding mechanism in a second embodiment;
FIG. 5B is a side view around a holding portion;
FIG. 5C is a perspective view around the holding portion;
FIG. 6 is a side view around a holding portion in a holding mechanism in Modification 1 of the second embodiment; and
FIG. 7 is a side view around a holding portion in a holding mechanism in Modification 2 of the second embodiment.

### Brief Description of Embodiments

Hereinafter, embodiments of the present invention will be described in detail with reference to the drawings.

### [First Embodiment]

### [Configuration]

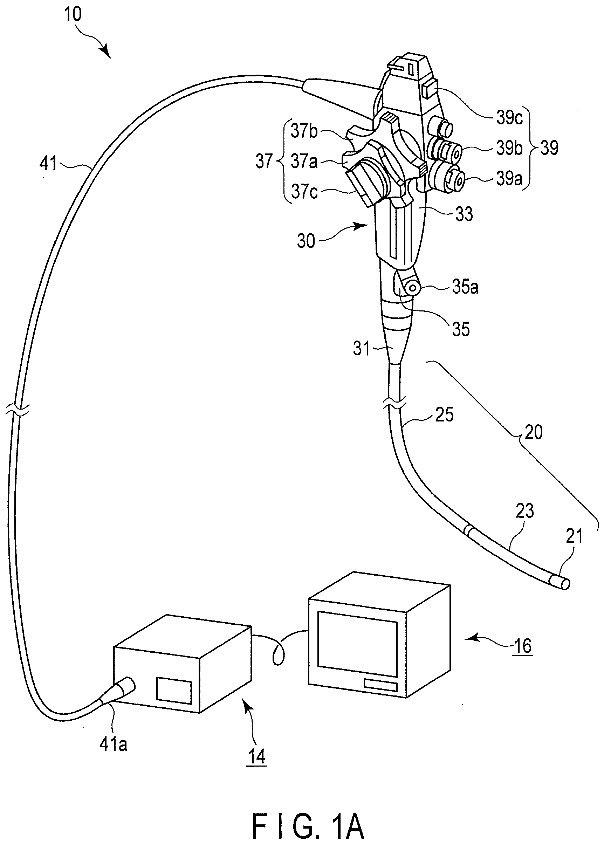

A first embodiment will be described with reference to FIG. 1A, FIG. 1B, FIG. 2A, FIG. 2B, FIG. 2C, FIG. 3A, FIG. 3B, FIG. 3C and FIG. 3D. It is to be noted that, for example, in FIG. 1A, drawing of a holding mechanism 500 for an endoscopic guide member is omitted, and in this way, part of the drawings omit part of members for clarification of the drawing.

### [Endoscope 10]

As shown in FIG. 1A, an endoscope 10 is, for example, a side view type of endoscope.

As shown in FIG. 1A, the endoscope 10 has a hollow elongated inserting section 20 to be inserted to a lumen such as a body cavity of a patient, and an operating section 30 that is coupled with a proximal end portion of the inserting section 20 and operates the endoscope 10.

### [Inserting Section 20]

As shown in FIG. 1A, the inserting section 20 has a distal end hard portion 21, a bending portion 23, and a flexible tube portion 25 from a distal end portion side of the inserting section 20 toward a proximal end portion side of the inserting section 20. A proximal end portion of the distal end hard portion 21 is coupled to a distal end portion of the bending portion 23, and a proximal end portion of the bending portion 23 is coupled to a distal end portion of the flexible tube portion 25.

The distal end hard portion 21 is a distal end portion of the inserting section 20, is hard, and does not bend. A configuration of the distal end hard portion 21 will be described later.

The bending portion 23 is bent in a desirable direction such as an upward, downward, right or left direction by an operation of a bending operation portion 37 which will be described later. When the bending portion 23 is bent, a position and an orientation of the distal end hard portion 21 change. Further, an observation object is illuminated with unshown illumination light, and the observation object is captured in an observation view field. This observation object is, e.g., an affected region, a lesion region or the like in a subject (e.g., a body cavity).

The flexible tube portion 25 has a desirable flexibility. Therefore, the flexible tube portion 25 bends by an external force. The flexible tube portion 25 is a tubular member extended from an after-mentioned main body portion 31 in the operating section 30.

### [Operating Section 30]

As shown in FIG. 1A, the operating section 30 has the main body portion 31 from which the flexible tube portion 25 extends, a grasping portion 33 which is coupled to a proximal end portion of the main body portion 31 and which is grasped by an operator who operates the endoscope 10, and a universal cord 41 connected to the grasping portion 33.

### [Grasping Portion 33]

As shown in FIG. 1A, the grasping portion 33 has a treatment instrument inserting portion 35, the bending operation portion 37 which is operated to bend the bending portion 23, and a switch portion 39. The treatment instrument inserting portion 35 is disposed on a distal end portion side of the grasping portion 33, and the bending operation portion 37 and the switch portion 39 are disposed on a proximal end portion side of the grasping portion 33.

### [Treatment instrument Inserting Portion 35]

As shown in FIG. 1A, the treatment instrument inserting portion 35 is branched to the grasping portion 33. Consequently, a central axis direction of the treatment instrument inserting portion 35 tilts to a central axis direction of the grasping portion 33.

As shown in FIG. 1A, the treatment instrument inserting portion 35 has a treatment instrument insertion port portion 35a which is disposed in an end portion of the treatment instrument inserting portion 35 and disposed so that a guide member 300 and a treatment instrument 400 described later are inserted to the endoscope 10.

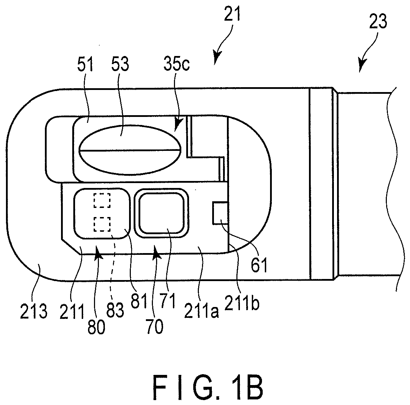

The treatment instrument insertion port portion 35a is coupled to a proximal end portion of an unshown treatment instrument inserting channel. The treatment instrument inserting channel is disposed inside the inserting section 20, and disposed along the distal end hard portion 21 from the flexible tube portion 25 via the bending portion 23. A distal end portion of the treatment instrument inserting channel communicates with a distal end opening portion 35c (see FIG. 1B) disposed in the distal end hard portion 21. The treatment instrument insertion port portion 35a is an insertion port through which the guide member 300 and the treatment instrument 400 are inserted to the treatment instrument inserting channel.

As shown in FIG. 1A, a central axis of the treatment instrument insertion port portion 35a is disposed coaxially with a central axis of the treatment instrument inserting portion 35, and therefore tilts to a central axis of the grasping portion 33. Furthermore, the central axis direction tilts to the central axis direction of the grasping portion 33.

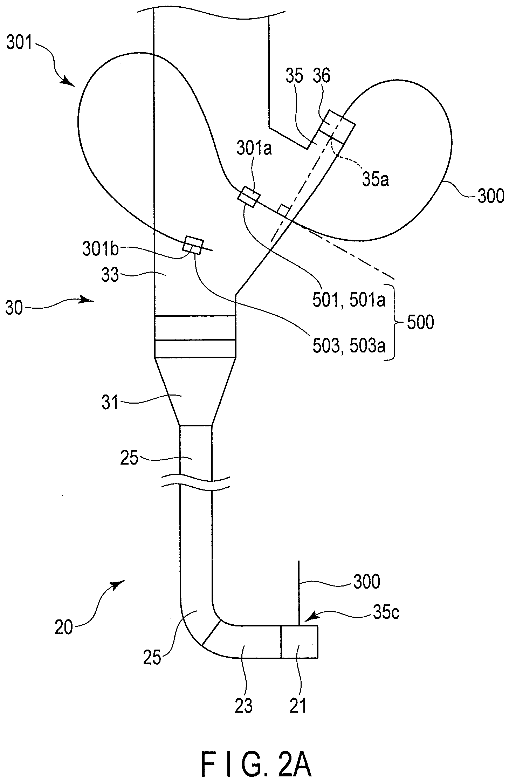

As shown in FIG. 2A, the treatment instrument inserting portion 35 further has a cylindrical forceps plug portion 36 disposed in the treatment instrument inserting portion 35. The forceps plug portion 36 is made of a resin such as a rubber. A central axis of the forceps plug portion 36 is disposed coaxially with the central axis of the treatment instrument insertion port portion 35a. Consequently, the forceps plug portion 36 tilts to the grasping portion 33. When the forceps plug portion 36 is disposed in the treatment instrument inserting portion 35, the forceps plug portion 36 communicates with the treatment instrument inserting channel via the treatment instrument insertion port portion 35a.

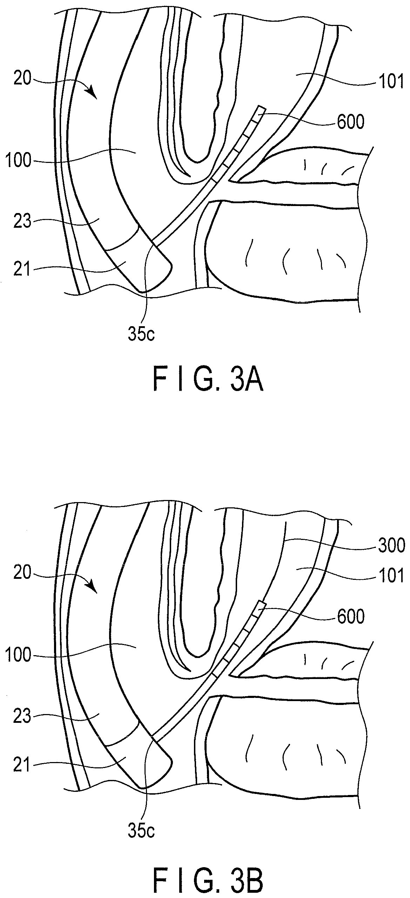

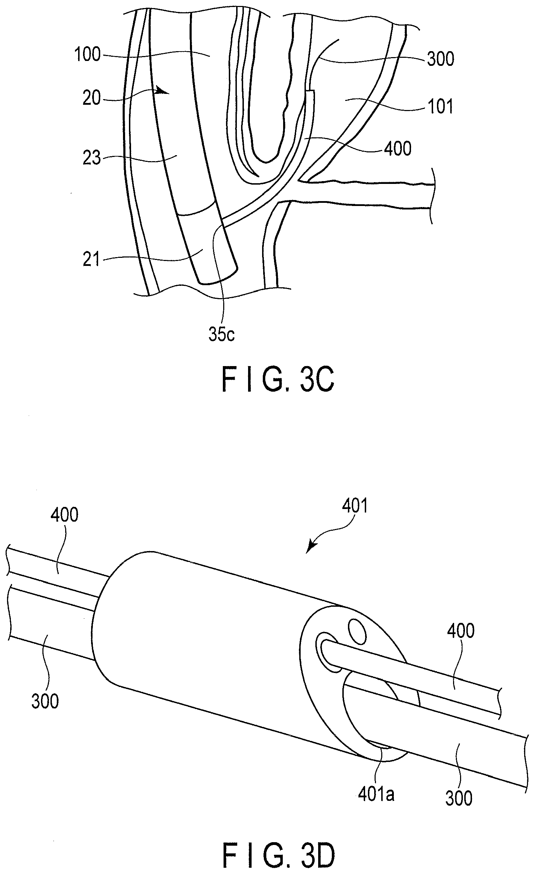

The guide member 300 shown in FIG. 2A and the treatment instrument 400 shown in FIG. 3C are inserted to the treatment instrument inserting channel from the forceps plug portion 36 via the treatment instrument insertion port portion 35a, and are pushed up to the distal end hard portion 21 side. Further, as shown in FIG. 2A, FIG. 3B and FIG. 3C, the guide member 300 and the treatment instrument 400 are projected from the distal end opening portion 35c.

### [Bending operation portion 37]

As shown in FIG. 1A, the bending operation portion 37 has a right/left bending operation knob 37a that which is operated to bend the bending portion 23 right and left, an upward/downward bending operation knob 37b which is operated to bend the bending portion 23 upward and downward, and a fixing knob 37c which fixes a position of the bending portion 23 which is bent.

### [Switch Portion 39]

As shown in FIG. 1A, the switch portion 39 has a suction switch 39a, a gas sending/water sending switch 39b, and various switches 39c for endoscope photography. The suction switch 39a, the gas sending/water sending switch 39b and the various switches 39c are operated by an operator's hand when the grasping portion 33 is grasped by the operator.

The suction switch 39a is operated when the endoscope 10 sucks mucus, fluid or the like from the distal end opening portion 35c that also serves as a suction opening portion via the treatment instrument inserting channel that also serves as a suction channel.

For the purpose of acquiring the observation view field of an imaging unit 70 in the distal end hard portion 21, the gas sending/water sending switch 39b is operated when a fluid is sent from an unshown gas sending tube and an unshown gas sending/water sending tube, and when the fluid is sent from the water sending tube and the gas sending/water sending tube. The fluid includes water or a gas.

The gas sending tube, the water sending tube and the gas sending/water sending tube are disposed from the inserting section 20 via the main body portion 31 and the grasping portion 33 up to the universal cord 41 inside the endoscope 10.

### [Universal Cord 41]

As shown in FIG. 1A, the universal cord 41 is extended from a side surface of the grasping portion 33. The universal cord 41 has a connecting connector 41a that is detachable from and attachable to a control device 14. The control device 14 controls the endoscope 10. The control device 14 has an image processing section that processes an image imaged by the imaging unit. The control device 14 is connected to a display section 16 that is a display section to display the image imaged by the imaging unit.

### [Configuration of Distal end hard Portion 21]

As shown in FIG. 1B, the distal end hard portion 21 has a main body portion 211 and a cap portion 213.

The main body portion 211 is made of a metal such as stainless steel. A proximal end portion of the main body portion 211 is coupled to the distal end portion of the bending portion 23. The main body portion 211 has, e.g., a columnar shape. Further, as shown in FIG. 1B, a part of an outer peripheral surface of the main body portion 211 is formed into a planar shape by cutting a part of the main body portion 211. In this way, the main body portion 211 has a flat surface portion 211a, and a wall surface portion 211b orthogonal to the flat surface portion 211a.

As shown in FIG. 1B, the cap portion 213 covers the main body portion 211 except the flat surface portion 211a and the wall surface portion 211b. The cap portion 213 is water-tightly bonded to the main body portion 211. The cap portion 213 is fixed to the main body portion 211. The cap portion 213 has an electric insulating property.

### [Main Body Portion 211]

As shown in FIG. 1B, in the main body portion 211, there are mounted the distal end opening portion 35c, a gas sending/water sending nozzle 61, the imaging unit 70, and an illuminating unit 80.

### [Distal End Opening portion 35c]

As shown in FIG. 1B, the distal end opening portion 35c is concaved to the flat surface portion 211a. That is, the distal end opening portion 35c opens toward a lateral side of the main body portion 211. The distal end opening portion 35c stores a treatment instrument raising base 51 that is remotely swingably operable in accordance with a raising operation on a hand side. The treatment instrument raising base 51 is connected to an unshown raising base operating section via an unshown raising operation wire into which the inserting section 20 is inserted. The raising base operating section is disposed in, e.g., the grasping portion 33 indicating the hand side. When the raising base operating section pushes and pulls the raising operation wire, the treatment instrument raising base 51 freely turns to the main body portion 211 around an unshown support shaft as a center. Further, for example, the treatment instrument raising base 51 swings between a storage position where the treatment instrument raising base 51 is inverted to the flat surface portion 211a of the main body portion 211, and is stored in the main body portion 211 and a rising position where the treatment instrument raising base 51 projects (rises) from the flat surface portion 211a of the main body portion 211, i.e., the distal end opening portion 35c. The treatment instrument raising base 51 is swung in accordance with the raising operation, thereby projecting and guiding the treatment instrument 400 from the distal end opening portion 35c to the lateral side of the main body portion 211. It is to be noted that the treatment instrument raising base 51 may have a treatment instrument guiding groove portion 53 that guides the treatment instrument 400 from the main body portion 211 to the outside. The treatment instrument guiding groove portion 53 is disposed in a central portion of the treatment instrument raising base 51, and further disposed along a longitudinal axis direction of the treatment instrument raising base 51.

### [Gas Sending/Water Sending Nozzle 61]

As shown in FIG. 1B, the gas sending/water sending nozzle 61 is disposed in the wall surface portion 211b of the main body portion 211. For example, the gas sending/water sending nozzle 61 and an observation window portion 71 of the imaging unit 70 are disposed on the same straight line in an axial direction of the inserting section 20 to send the gas or send the water to the observation window portion 71 of the imaging unit 70. The gas sending/water sending nozzle 61 including the wall surface portion 211b is disposed closer to a proximal end portion side of the main body portion 211 than the observation window portion 71. The gas sending/water sending nozzle 61 is connected to the gas sending tube and the water sending tube via the unshown gas sending/water sending tube inserted into inside the inserting section 20. By the operation of the gas sending/water sending switch 39b, the gas sending/water sending nozzle 61 sends the gas or sends the water to the observation window portion 71.

### [Imaging Unit 70]

As shown in FIG. 1B, the imaging unit 70 has the observation window portion 71, an unshown prism portion, and an unshown imaging section.

### [Observation Window Portion 71]

As shown in FIG. 1B, reflected light reflected from the observation object enters into the observation window portion 71 in side viewing observation. Consequently, the observation window portion 71 is disposed in a peripheral surface of the main body portion 211, e.g., the flat surface portion 211a, and disposed on the same flat surface as the flat surface portion 211a. That is, the imaging unit 70 is embedded in the main body portion 211 so that the observation window portion 71 is disposed in the flat surface portion 211a. The observation window portion 71 is exposed from the cap portion 213. A central axis of the observation window portion 71 is disposed orthogonally to a central axis of the main body portion 211.

### [Prism Portion]

The unshown prism portion reflects the reflected light transmitted through the observation window portion 71, so that traveling of the reflected light can vary. The prism portion is disposed inside the main body portion 211 so that a central axis of the prism portion is substantially disposed coaxially with the central axis of the observation window portion 71. The prism portion is disposed inside the main body portion 211, to face the observation window portion 71 in an orthogonal direction.

### [Imaging Section]

The unshown imaging section is disposed adjacent to the prism portion in the axial direction of the inserting section 20. That is, the imaging section and the prism portion are substantially disposed on the same straight line in the axial direction of the inserting section 20. The imaging section is disposed together with the prism portion along the axial direction of the inserting section 20. Such an imaging section is disposed inside the main body portion 211, to face the prism portion.

The imaging section has an unshown objective optical system (lens system) having an objective lens group having a predetermined image surface distortion, an unshown imaging element such as a CCD disposed at an image forming position of the objective optical system, and an unshown connecting circuit substrate. The objective optical system (the lens system), the imaging element and the connecting circuit substrate are integrated.

The connecting circuit substrate is connected to an unshown imaging cable such as a signal line. The imaging cable is inserted into the connecting connector 41a via the bending portion 23, the flexible tube portion 25, the operating section 30 and the universal cord 41. Thus, the connecting connector 41a is connected to the control device 14, and hence, the imaging cable is connected to the control device 14, and the observation object imaged by the imaging unit 70 is displayed in the display section 16.

It is to be noted that in the objective optical system, at least part of the lenses may be movable along a longitudinal axis C direction. Consequently, the imaging element can image the observation object in a state where the image of the observation object is focused on the imaging element.

In place of the imaging element, a distal end portion of an unshown image guide fiber may be fixed, thus the endoscope is not limited to an electron scope but may be a fiber scope.

### [Illuminating Unit 80]

As shown in FIG. 1B, the illuminating unit 80 has an illuminating window portion 81, an illuminating section 83, and an unshown illuminating cable to be connected to the illuminating section 83. The illuminating unit 80 is disposed as a separate unit from the imaging unit 70.

### [Illuminating Window Portion 81]

As shown in FIG. 1B, the illuminating window portion 81 transmits the illumination light with which the observation object is to be irradiated, in the side viewing observation. Consequently, the illuminating window portion 81 is disposed in the peripheral surface of the main body portion 211, e.g., the flat surface portion 211a, and is disposed on the same flat surface as the flat surface portion 211a. That is, the illuminating unit 80 is embedded in the main body portion 211 so that the illuminating window portion 81 is disposed in the flat surface portion 211a. The illuminating window portion 81 is exposed from the cap portion 213. The illuminating window portion 81 is disposed adjacent to the observation window portion 71 in the axial direction of the inserting section 20. That is, the illuminating window portion 81 and the observation window portion 71 are disposed on the same straight line in the axial direction of the inserting section 20. The illuminating window portion 81 is disposed closer to a distal end portion side of the main body portion 211 than, e.g., the observation window portion 71. A central axis of the illuminating window portion 81 is disposed along the orthogonal direction.

### [Illuminating Section 83]

The illuminating section 83 emits illumination light such as white light toward, e.g., the upside that is the lateral side. The illuminating section 83 has, e.g., an illumination light emitting end portion such as a light guide that guides the illumination light emitted from a light source, or a light emitting element such as an LED. For example, two illuminating sections 83 are disposed.

As shown in FIG. 1B, the illuminating section 83 is disposed inside the main body portion 211 so that a central axis of the illuminating section 83 is substantially disposed coaxially with the central axis of the illuminating window portion 81. The illuminating section 83 is disposed below the illuminating window portion 81. The illuminating section 83 is disposed inside the main body portion 211, to face the illuminating window portion 81 in the orthogonal direction.

### [Illuminating Cable]

The unshown illuminating cable is disposed in each illuminating section 83. The illuminating cable has a longitudinal axis opposite to a planar direction of an upper surface portion 85a. This longitudinal axis is disposed in parallel with the axial direction of the inserting section 20. The illuminating cable is disposed along the axial direction of the inserting section 20, and disposed lower than the illuminating section 83 in the orthogonal direction orthogonal to the axial direction of the inserting section 20. The illuminating cable is inserted into the connecting connector 41a via the bending portion 23, the flexible tube portion 25, the operating section 30 and the universal cord 41. By connecting the connecting connector 41a to the control device 14, the illuminating cable is connected to the control device 14, and a power to emit the illumination light from the illuminating section 83 is supplied to the illuminating cable. Further, the illuminating cable supplies the power to the illuminating section 83.

### [Endoscopic Guide Member 300 and Treatment instrument 400 and Holding Mechanism 500 for Guide Member 300]

As shown in FIG. 2A, FIG. 3A, FIG. 3B, FIG. 3C and FIG. 3D, the endoscope 10 is used together with the endoscopic guide member 300 and the treatment instrument 400 which are separate members from the endoscope 10. The endoscope 10 further has the holding mechanism 500 holding the guide member 300 that is inserted from the forceps plug portion 36 (the treatment instrument insertion port portion 35a) to the treatment instrument inserting channel inside the endoscope 10 and projects from the distal end opening portion 35c before the treatment instrument 400, thereby reaching the subject, and that guides the treatment instrument 400 to the subject.

### [Guide Member 300]

As shown in FIG. 2A and as described above, the guide member 300 is inserted to the treatment instrument inserting channel from the forceps plug portion 36 via the treatment instrument insertion port portion 35a. A distal end portion of the guide member 300 is inserted into the treatment instrument inserting channel, and projects from the distal end opening portion 35c disposed in a side surface of the distal end portion of the inserting section 20. The distal end opening portion 35c is disposed in the side surface, and hence, the distal end portion of the guide member 300 projects to a lateral side of the distal end portion of the inserting section 20. The distal end portion of the guide member 300 reaches the subject. In this way, the guide member 300 is inserted to the lumen through the inserting section 20 of the endoscope 10 which is inserted to the lumen. Further, the guide member 300 projects from the distal end opening portion 35c of the inserting section 20, and guides the treatment instrument 400. It is to be noted that a proximal end portion 301 of the guide member 300 is exposed from the forceps plug portion 36 to the outside of the endoscope 10 in a state where the distal end portion of the guide member 300 is projected from the distal end opening portion 35c to the outside.

The guide member 300 is inserted from the forceps plug portion 36 to the treatment instrument inserting channel, before the treatment instrument 400 is inserted from the forceps plug portion 36 to the treatment instrument inserting channel. Details will be described later, but the guide member 300 guides the treatment instrument 400 to the subject in a state where the guide member 300 is inserted to the treatment instrument inserting channel and the distal end portion of the guide member 300 is projected from the distal end opening portion 35c to the outside.

The guide member 300 is formed by, e.g., a fine linear member. The linear member includes, e.g., a wire made of stainless steel. The linear member may include, e.g., a nickel titanium wire. The surface of the nickel titanium wire is coated with, e.g., fluororesin or the like.

### [Treatment instrument 400]

As shown in FIG. 3C, the treatment instrument 400 is used to treat a subject such as a bile duct 101. The treatment instrument 400 has, e.g., a knife for endoscopic sphincterotomy (hereinafter, EST), or the like. The treatment instrument 400 is formed by, e.g., a fine linear member.

As shown in FIG. 3D, the treatment instrument 400 is used together with a cylinder member 401. The cylinder member 401 functions as, e.g., a multilumen, and has an insertion port portion 401a into which the guide member 300 can be inserted, and an engagement port portion with which, e.g., a distal end portion of the treatment instrument 400 engages. The cylinder member 401 is thicker than holding portions 501 and 503 which will be described later. The cylinder member 401 is slidable along the guide member 300 so that the guide member 300 is inserted into the insertion port portion 401a. By this sliding, the cylinder member 401 functions as a monorail portion, and the treatment instrument 400 is movable along the guide member 300. At this time, the treatment instrument 400 is guided by the guide member 300 and is inserted to the treatment instrument inserting channel from the forceps plug portion 36 via the treatment instrument insertion port portion 35a. Further, the treatment instrument 400 is guided by the guide member 300 to be inserted into the treatment instrument inserting channel, and projects from the distal end opening portion 35c to the lateral side to reach the subject.

In this way, the treatment instrument 400 is guided to the subject by the guide member 300.

### [Holding Mechanism 500]

In general, the treatment instrument 400 is operated so that the treatment instrument 400 moves forward and backward along an axial direction of the treatment instrument 400 by an operation of an operator's right hand in a state where the grasping portion 33 is grasped with an operator's left hand. Consequently, the holding mechanism 500 is disposed in the operating section 30 so that the holding mechanism 500 can hold the proximal end portion 301 of the guide member 300 or release the holding by the operation of the operator's right hand in the state where the grasping portion 33 is grasped with the operator's left hand. In other words, the holding mechanism 500 is disposed in the operating section 30 so that the proximal end portion 301 of the guide member 300 is attachable to or detachable from the grasping portion 33 via the holding mechanism 500 by the operation of the operator's right hand in the state where the grasping portion 33 is grasped with the operator's left hand. In this way, the holding mechanism 500 is disposed in the operating section 30 so that the operation of the left hand of the operator who operates the endoscope 10 is not disturbed and so that the holding mechanism 500 can be operated by the operation of the right hand. The holding mechanism 500 is disposed on, e.g., the same straight line as that of a distal end portion of the grasping portion 33 and the bending operation portion 37.

As shown in FIG. 2A, FIG. 3B and FIG. 3C, the holding mechanism 500 disposed in this way holds the guide member 300 that reaches the subject to guide the treatment instrument 400 to the subject as described above, when the treatment instrument 400 is inserted to the treatment instrument inserting channel inside the endoscope 10 from the forceps plug portion 36 disposed in the operating section 30 of the endoscope 10 via the treatment instrument insertion port portion 35a, and projects from the distal end opening portion 35c disposed in the distal end portion of the inserting section 20 of the endoscope 10 to treat the subject. Specifically, as shown in FIG. 2A, the holding mechanism 500 holds the proximal end portion 301 of the guide member 300 which is exposed from the forceps plug portion 36 to the outside of the endoscope 10 so that the guide member 300 is fixed and positional shift of the guide member 300 is prevented.

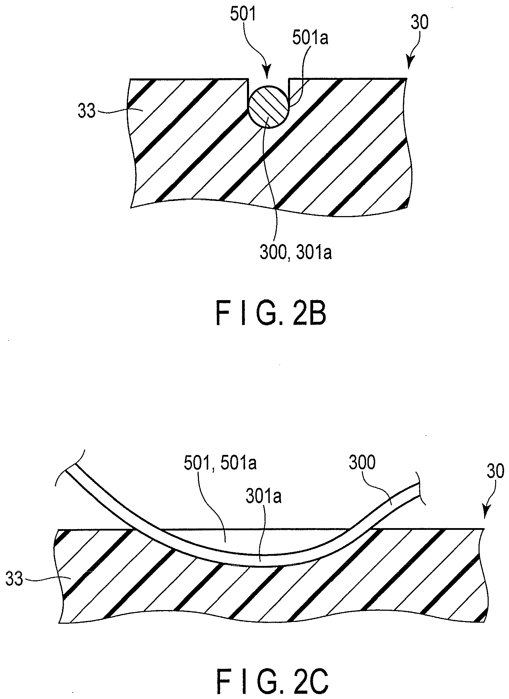

Thus, as shown in FIG. 2A, FIG. 2B and FIG. 2C, the holding mechanism 500 has the holding portion 501 holding a part 301a of the proximal end portion 301 of the guide member 300, and the holding portion 503 holding another part 301b of the proximal end portion 301 of the guide member 300 which is a portion other than the one part 301a held by the holding portion 501. That is, the holding portion 501 and the holding portion 503 hold mutually different parts of the guide member 300. In the present embodiment, for example, the one part 301a is disposed closer to a distal end portion side of the guide member 300 than the other part 301b. In this way, the holding portion 501 holds the portion other than the proximal end portion 301 of the guide member 300 which is held by the holding portion 503. The holding portion 501 is a separate portion from the holding portion 503, and is disposed at a position different from that of the holding portion 503.

That is, the holding portion 501 is disposed in the operating section 30 that operates the endoscope 10 and that is coupled to the proximal end portion of the inserting section 20. The holding portion 501 holds, in the operating section 30, the one part 301a which is one part exposed from the endoscope 10 on a proximal side of the guide member 300 in a state where the distal end portion of the guide member 300 is projected from the distal end opening portion 35c.

The holding portion 503 is disposed at the position different from that of the holding portion 501 in the operating section 30. As described above, the holding portion 503 holds the other part 301b of the guide member 300 at the position different from that of the part held by the holding portion 501 on the proximal side of the guide member 300 and in an orientation different from an orientation of the holding portion 501. The holding portions 501 and 503 are disposed in the operating section 30. In other words, the operating section 30 holds the mutually different parts of the guide member 300 on the proximal side thereof at the positions different from each other and in the orientations different from each other in the operating section 30.

### [Holding Portion 501]

As shown in FIG. 2A, FIG. 2B and FIG. 2C, the holding portion 501 is disposed in the operating section 30 so that the holding portion 501 is integrated with an exterior portion of the operating section 30 of the endoscope 10, e.g., an exterior portion of the grasping portion 33. As shown in FIG. 2A, the holding portion 501 holds the one part 301a exposed from the forceps plug portion 36 to the outside of the endoscope 10 so that the guide member 300 is fixed and the positional shift of the guide member 300 is prevented in a state where the distal end portion of the guide member 300 is projected from the distal end opening portion 35c. The holding portion 501 holds the one part 301a so that the one part 301a is attachable to and detachable from the operating section 30. That is, the holding portion 501 can release the holding of the one part 301a. In other words, the one part 301a can be removed from the operating section 30.

As shown in FIG. 2A, for example, the holding portion 501 holds the one part 301a so that an axial direction of the one part 301a exposed to the outside of the endoscope 10 is orthogonal to an axial direction (an opening direction) of the treatment instrument insertion port portion 35a, and for this purpose, the holding portion 501 is disposed so that an axial direction of the holding portion 501 is along the orthogonal direction orthogonal to the axial direction of the treatment instrument insertion port portion 35a. The holding portion 501 is disposed on, e.g., an axis different from the central axis of the treatment instrument insertion port portion 35a.

As shown in FIG. 2A, for example, the holding portion 501 is disposed close to the treatment instrument insertion port portion 35a. In detail, the holding portion 501 is disposed between the treatment instrument insertion port portion 35a and the proximal end portion of the inserting section 20, specifically the proximal end portion of the main body portion 31 in an axial direction of the operating section 30. The holding portion 501 may be disposed in, e.g., the exterior portion of the grasping portion 33 or an exterior portion of the treatment instrument inserting portion 35.

### [Holding Portion 503]

As shown in FIG. 2A, the holding portion 503 is disposed at the position different from that of the holding portion 501. As shown in FIG. 2A, the holding portion 503 holds the other part 301b exposed from the forceps plug portion 36 to the outside of the endoscope 10, in the orientation different from the orientation of the holding portion 501 so that the guide member 300 is fixed and the positional shift of the guide member 300 is prevented, in the state where the distal end portion of the guide member 300 is projected from the distal end opening portion 35c. The orientation of the holding portion 501 includes an orientation in which the holding portion 501 holds the one part 301a. As described above, the other part 301b to be held by the holding portion 503 is the portion other than the one part 301a to be held by the holding portion 501. The holding portion 503 holds the other part 301b so that the other part 301b is attachable to and detachable from the operating section 30. That is, the holding portion 503 can release the holding of the other part 301b. In other words, the other part 301b can be removed from the operating section 30.

As shown in FIG. 2A, for example, the holding portion 503 is disposed away from the treatment instrument inserting portion 35. In detail, the holding portion 501 is disposed between the holding portion 501 and the proximal end portion of the inserting section 20, specifically the proximal end portion of the main body portion 31 in the axial direction of the operating section 30. The holding portion 503 is disposed in the operating section 30, to be integrated with the exterior portion of the operating section 30, e.g., the exterior portion of the grasping portion 33.

According to the above description, the holding portion 503 is disposed at the position different from that of the holding portion 501, and holds the part different from the part held by the holding portion 501. The holding portion 503 of the present embodiment holds the proximal end portion 301 of the guide member 300 passed through the holding portion 501.

For example, the holding portion 503 holds the other part 301b so that an axial direction of the other part 301b exposed to the outside of the endoscope 10 is orthogonal to an axial direction of the grasping portion 33 of the operating section 30, and for this purpose, the holding portion 503 is disposed so that an axial direction of the holding portion 503 is along an orthogonal direction orthogonal to the axial direction of the grasping portion 33. Consequently, the holding portion 503 is disposed so that, e.g., the axial direction of the holding portion 503 is along a direction different from the axial direction of the holding portion 501. In other words, the holding portion 503 holds the guide member 300 in the orientation different from that of the holding portion 501.

### [One Example of Configuration of Holding Portions 501 and 503]

The holding portion 501 and the holding portion 503 have the same configuration, and hence, the holding portion 501 will be described.

As shown in FIG. 2B and FIG. 2C, the holding portion 501 has, e.g., a groove portion 501a which is formed so that a part of a planar exterior portion of the operating section 30 is notched toward the inside of the exterior portion and with which the one part 301a is engageable. The groove portion 501a has, for example, a U-shape or a concave shape. An inner diameter of the groove portion 501a is substantially the same as an outer diameter of the guide member 300, and an inner shape of the groove portion 501a is substantially the same as an outer shape of the guide member 300, so that the groove portion 501a engages with the guide member 300. In consequence, the guide member 300 engages with the groove portion 501a, whereby the holding portion 501 holds the guide member 300, and the holding portion 501 fixes the proximal end portion 301 of the guide member 300 to the operating section 30. It is to be noted that a groove portion in the holding portion 503 will be referred to as a groove portion 503a. It is to be noted that at least one of the holding portion 501 and the holding portion 503 may have the groove portion.

### [Operation]

For example, in a case where a subject such as the bile duct 101 is treated by the treatment instrument 400, e.g., the EST knife or the like, as shown in FIG. 3A, the inserting section 20 is inserted to a duodenum 100. Next, a contrasting tube 600 is inserted to the bile duct 101 from the forceps plug portion 36 via the treatment instrument insertion port portion 35a, the treatment instrument inserting channel, the treatment instrument raising base 51 and the distal end opening portion 35c. Further, a contrast media is injected in the bile duct 101 via the contrasting tube 600.

Next, the guide member 300 is inserted to the treatment instrument inserting channel from the forceps plug portion 36 via the treatment instrument insertion port portion 35a. Further, as shown in FIG. 3B, the distal end portion of the guide member 300 is inserted into the treatment instrument inserting channel, and projects from the distal end opening portion 35c. The distal end opening portion 35c is disposed in the side surface, and hence, the distal end portion of the guide member 300 projects to the lateral side of the distal end portion of the inserting section 20. Further, the distal end portion of the guide member 300 reaches a subject such as the bile duct 101. As shown in FIG. 2A, the proximal end portion 301 of the guide member 300 is exposed from the forceps plug portion 36 to the outside of the endoscope 10. Further, the contrasting tube 600 is pulled outside.

In the above description, the guide member 300 is inserted by the operation of the operator's right hand in the state where the grasping portion 33 is grasped with the operator's left hand. The contrasting tube 600 is pulled outside by the operation of the operator's right hand in the state where the grasping portion 33 is grasped with the operator's left hand.

Next, as shown in FIG. 3C and FIG. 3D, the treatment instrument 400 moves along the guide member 300 so that the guide member 300 passes through the cylinder member 401 functioning as the monorail portion. At this time, the treatment instrument 400 is guided by the guide member 300 and is inserted to the treatment instrument inserting channel from the forceps plug portion 36 via the treatment instrument insertion port portion 35a. Further, the treatment instrument 400 is guided by the guide member 300 to be inserted into the treatment instrument inserting channel, and projects from the distal end opening portion 35c to the lateral side to reach the subject.

In this way, the treatment instrument 400 is guided to the subject by the guide member 300.

In the above description, the treatment instrument 400 is inserted by the operation of the operator's right hand in the state where the grasping portion 33 is grasped with the operator's left hand.

It is to be noted that although not shown, a proximal end portion of the treatment instrument 400 is exposed from the forceps plug portion 36 to the outside of the endoscope 10 in the same manner as in the proximal end portion 301 of the guide member 300.

Next, as shown in FIG. 2A, FIG. 2B and FIG. 2C, the holding mechanism 500 holds the proximal end portion 301 of the guide member 300 at two positions of the holding portions 501 and 503. At this time, the proximal end portion 301 of the guide member 300 is pulled out from the forceps plug portion 36, then reaches from the forceps plug portion 36 toward the holding portion 501, for example, to draw, e.g., a loop, and further reaches from the holding portion 501 toward the holding portion 503, for example, to draw, e.g., a loop. For example, the holding portion 501 first holds the one part 301a, and next the holding portion 503 holds the other part 301b. This order is not especially limited.

Specifically, the proximal end portion 301 of the guide member 300 engages with the groove portions 501a and 503a by the operation of the operator's right hand in the state where the grasping portion 33 is grasped with the operator's left hand. That is, the guide member 300 is attached to the operating section 30. In consequence, the guide member 300 is fixed, and the positional shift of the guide member 300 is prevented.

In the above description, the guide member 300 is attached to the operating section 30 via the holding mechanism 500 by the operation of the operator's right hand in the state where the grasping portion 33 is grasped with the operator's left hand.

Next, in this state, the treatment instrument 400 treats the subject. When the treatment of the treatment instrument 400 ends, the treatment instrument 400 is pulled out from the endoscope 10 in a state where the guide member 300 is left.

At this time, as described above, the holding mechanism 500 holds the proximal end portion 301 of the guide member 300 at two positions of the holding portions 501 and 503. The holding portion 501 and the holding portion 503 are disposed at positions different from each other, and hold different parts of the guide member 300 from each other. Consequently, the guide member 300 is fixed to the operating section 30, and the positional shift of the guide member 300 due to a pulling operation of the treatment instrument 400 is prevented.

When the treatment instrument 400 is pulled outside, it is possible that the guide member 300 will be pulled outside by this force. However, in the present embodiment, the holding portion 501 holds the one part 301a, whereby the axial direction of the one part 301a is orthogonal to the axial direction of the treatment instrument insertion port portion 35a. Consequently, even when the abovementioned force is generated, the position of the guide member 300 is prevented from noticeably shifting.

When the treatment instrument 400 is pulled outside, a force accompanying the pulling is exerted from the forceps plug portion 36 toward the upside, i.e., the operating section 30. However, in the present embodiment, the holding portions 501 and 503 are disposed lower than the treatment instrument insertion port portion 35a. Specifically, the holding portion 501 is disposed between the treatment instrument insertion port portion 35a and the proximal end portion of the main body portion 31 in the axial direction of the operating section 30, and the holding portion 503 is disposed between the holding portion 501 and the proximal end portion of the main body portion 31 in the axial direction of the operating section 30. Consequently, even when the abovementioned force is exerted toward the upside, the positional shift of the guide member 300 is prevented.

It is to be noted that when the cylinder member 401 functioning as the monorail portion and thicker than the treatment instrument 400 is pulled out from the forceps plug portion 36 and reaches the holding portion 501, the holding portion 501 releases the holding of the one part 301a once. Consequently, the cylinder member 401 can pass the holding portion 501. When the cylinder member 401 passes the holding portion 501, the holding portion 501 holds the one part 301a again. Next, when the cylinder member 401 reaches the holding portion 503, the holding portion 503 releases the holding of the other part 301b once. Consequently, the cylinder member 401 can pass the holding portion 503. When the cylinder member 401 passes the holding portion 503, the holding portion 503 holds the other part 301b again. Consequently, the treatment instrument 400 is pulled out from the endoscope 10, and the treatment instrument 400 is also removed from the guide member 300. In the above description, the guide member 300 is attached to or detached from the operating section 30 via the holding mechanism 500 by the operation of the operator's right hand in the state where the grasping portion 33 is grasped with the operator's left hand. The guide member 300 remains to be detained inside the endoscope 10 and inside a body.

Next, the holding mechanism 500 releases the holding of the proximal end portion 301 of the guide member 300 at two positions of the holding portions 501 and 503. For example, the holding portion 501 first releases the proximal end portion 301 of the guide member 300, and next the holding portion 503 releases the proximal end portion 301 of the guide member 300. This order is not especially limited. It is to be noted that the guide member 300 is removed from the operating section 30 by the operation of the operator's right hand in the state where the grasping portion 33 is grasped with the operator's left hand.

Next, an unshown basket is guided by the guide member 300 in the same manner as in the treatment instrument 400, and is inserted to the treatment instrument inserting channel from the forceps plug portion 36 via the treatment instrument insertion port portion 35a. Further, the basket is guided by the guide member 300 to be inserted into the treatment instrument inserting channel, and projects from the distal end opening portion 35c to the lateral side to reach the subject. Further, as described above, the holding mechanism 500 holds the proximal end portion 301 of the guide member 300 at two positions of the holding portions 501 and 503 again. Further, the basket collects a gallstone or the like. The basket is pulled out from the endoscope 10.

When the basket is pulled out from the forceps plug portion 36 and discharged to the outside of the endoscope 10, similarly to the above description, the holding mechanism 500 releases the holding of the proximal end portion 301 of the guide member 300 at two positions of the holding portions 501 and 503. Further, the basket is also removed from the guide member 300. Further, the guide member 300 is pulled out from the endoscope 10.

### [Effect]

In the present embodiment, the holding mechanism 500 is integral with the operating section 30, and hence, time and labor in attaching the holding mechanism 500 to the operating section 30 can be suppressed. That is, in the present embodiment, it is possible to omit the time and labor in attaching the holding mechanism 500 as a separate mechanism from the operating section 30 to, for example, the treatment instrument insertion port portion 35a. In the present embodiment, the holding mechanism 500 is integral with the operating section 30, and hence, the holding mechanism 500 can be inexpensive.

In the present embodiment, when the treatment instrument 400 is pulled outside, the guide member 300 is fixed and the positional shift of the guide member 300 is prevented by the holding mechanism 500. Therefore, in the present embodiment, the guide member 300 can securely be prevented from positional shifting by the holding mechanism 500. Consequently, in the present embodiment, the treatment instrument 400 can securely be guided to the subject by the guide member 300 in any situation.

In particular, the holding mechanism 500 holds the proximal end portion 301 of the guide member 300 at two positions of the holding portions 501 and 503. The holding portion 501 and the holding portion 503 are disposed at the positions different from each other, hold the portions of the guide member 300 which are different from each other, and hold the portions in the orientations different from each other. Consequently, in the present embodiment, the guide member 300 can be fixed to the main body portion 31, and the positional shift of the guide member 300 due to the pulling operation of the treatment instrument 400 can be prevented.

In the present embodiment, when the treatment instrument 400 is pulled outside, a force is generated in the axial direction of the treatment instrument insertion port portion 35a. In the present embodiment, when the holding portion 501 holds the one part 301a, the axial direction of the one part 301a is orthogonal to the axial direction of the treatment instrument insertion port portion 35a. Consequently, even when the abovementioned force is generated, the position of the guide member 300 can be prevented from noticeably shifting.

In the present embodiment, when the treatment instrument 400 is pulled outside, the force accompanying the pulling is exerted from the forceps plug portion 36 toward the upside, i.e., the operating section 30. However, in the present embodiment, the holding portions 501 and 503 are disposed lower than the treatment instrument insertion port portion 35a. Specifically, the holding portion 501 is disposed between the treatment instrument insertion port portion 35a and the proximal end portion of the main body portion 31 in the axial direction of the operating section 30, and the holding portion 503 is disposed between the holding portion 501 and the proximal end portion of the main body portion 31 in the axial direction of the operating section 30. Consequently, in the present embodiment, even when the abovementioned force is exerted toward the upside, the positional shift of the guide member 300 can be prevented.

In the present embodiment, the holding mechanism 500 can be disposed so that the proximal end portion 301 of the guide member 300 can be attached to or detached from the main body portion 31 by the operation of the operator's right hand in the state where the grasping portion 33 is grasped with the operator's left hand. Further, in the present embodiment, the operation of the left hand of the operator who operates the endoscope 10 can be prevented from being disturbed by the holding mechanism 500.

In the present embodiment, the holding portions 501 and 503 have the groove portions 501a and 503a, and hence, the proximal end portion 301 of the guide member 300 can easily be attached to or detached from the main body portion 31.

It is to be noted that, for example, in a case where a conventional holding mechanism different from the holding mechanism 500 of the present embodiment is embed in the distal end hard portion 21 in the vicinity of the distal end opening portion 35c, the distal end hard portion 21 thickens. In this case, a holding state of the conventional holding mechanism cannot visually be confirmed.

It is defined that a holding portion of the conventional holding mechanism functions as the groove portion 501a disposed in the treatment instrument raising base 51. In a case where a diameter of one guide member 300 is different from a diameter of the other guide member 300 for replacement, the holding portion might not securely hold both the guide members. In a case where the distal end portion of the guide member 300 has a tapered shape, the holding portion might not securely hold the guide member 300.

It is defined that the holding portion functions as the groove portion disposed in the treatment instrument raising base 51. When the treatment instrument 400 is pulled outside in a state where the guide member 300 is detained, the cylinder member 401 functioning as the monorail portion needs to pass the holding portion functioning as the groove portion. In this case, the holding portion needs to release the holding of the guide member 300, but there is the possibility that the guide member 300 shifts due to the releasing.

However, in the present embodiment, the abovementioned problem can be eliminated.

In the present embodiment, the holding mechanism 500 is exposed to the outside, and hence, the holding mechanism 500 can be cleaned together with the endoscope 10.

It is to be noted that in the present embodiment, when a central axis of the holding portion 501 is not substantially disposed coaxially with a central axis of the holding portion 503, the holding portion 503 may be disposed so that the axial direction of the holding portion 503 is along an orthogonal direction orthogonal to the axial direction of the treatment instrument insertion port portion 35a in the same manner as in the holding portion 501.

That is, at least one of the holding portion 501 and the holding portion 503 may be disposed to hold the guide member in a direction orthogonal to the axial direction of the treatment instrument insertion port portion 35a which the guide member 300 is inserted to the endoscope 10.

At least one of the holding portion 501 and the holding portion 503 may be disposed so that an axis of the one holding portion is along the orthogonal direction orthogonal to the axial direction of the operating section 30.

At least one of the holding portion 501 and the holding portion 503 may be disposed in the operating section 30 so that the one holding portion is integrated with the exterior portion of the operating section 30.

In the present embodiment, the inner diameter of the groove portion 501a is substantially the same as the outer diameter of the guide member 300 so that the groove portion 501a engages with the guide member 300, but the present invention does not have to be limited to this embodiment. For example, the inner diameter of the groove portion 501a may be minutely smaller than the outer diameter of the guide member 300 so that the groove portion 501a engages with the guide member 300. In consequence, a holding force of the holding portion 501 can be heightened. This aspect also similarly applies to the holding portion 503.

In the present embodiment, a part of the planar exterior portion of the operating section 30 is notched to form the groove portion 501a of the holding portion 501, but the present invention does not have to be limited to this embodiment. A raised part of the exterior portion may be notched to form the groove portion 501a. This aspect also similarly applies to the groove portion 503a. That is, at least one of the holding portion 501 and the holding portion 503 may have a U-shaped projecting portion that projects from the outer surface of the operating section 30.

The groove portion 501a may be covered with an unshown cover member in a state where the proximal end portion 301 of the guide member 300 is held. This aspect also similarly applies to the groove portion 503a.

In the present embodiment, the holding portion 501 has the groove portion 501a, but the present invention does not have to be limited to this embodiment. For example, the holding portion 501 may have an insertion hole portion into which the guide member 300 can be inserted, as long as the holding portion 501 can hold the guide member 300. This aspect also similarly applies to the holding portion 503.

In the present embodiment, the endoscope 10 is the side view type, but the endoscope 10 may be a direct view type.

In the present embodiment, at least one of the holding portion 501 and the holding portion 503 may have an elastic body which holds the guide member 300 so that the one holding portion is integrated with, e.g., the exterior portion of the grasping portion 33 of the operating section 30. In this case, the holding mechanism 500 may be formed of an elastic body such as a resin (e.g., integral forming).

### [Modification 1]

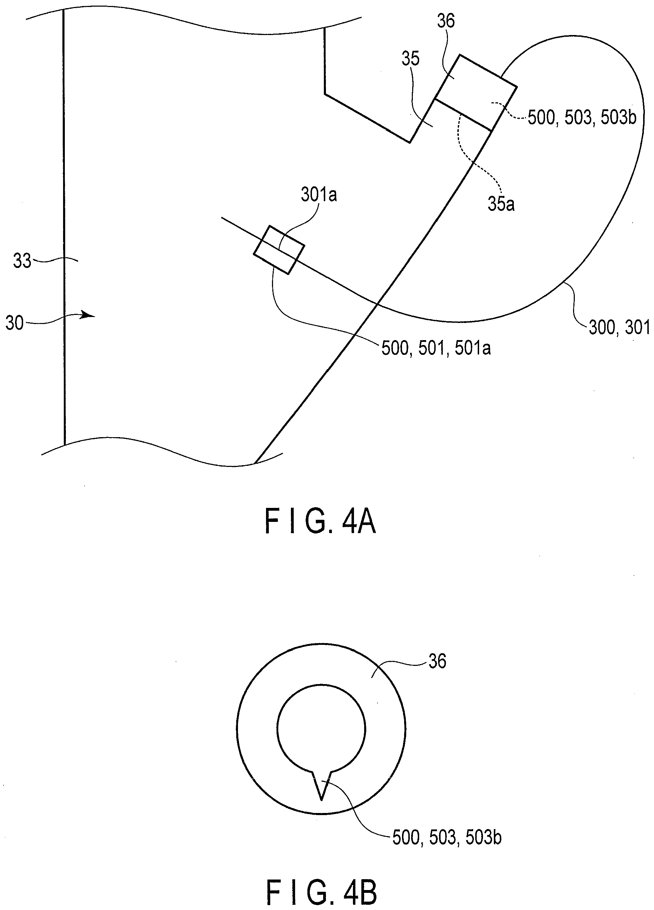

As shown in FIG. 4A and FIG. 4B, in the present modification, a holding portion 503 is disposed in an inner peripheral surface of a forceps plug portion 36 inserted to a treatment instrument insertion port portion 35a. For example, in the inner peripheral surface, the holding portion 503 is disposed at a position most away from a central axis of a grasping portion 33. The holding portion 503 has a groove portion 503b which is disposed along a longitudinal axis direction of the forceps plug portion 36 and with which another part 301b is engageable. The groove portion 503b is formed into a tapered shape from the inner peripheral surface of the forceps plug portion 36 toward an outer peripheral surface of the forceps plug portion 36 in a radial direction of the forceps plug portion 36. The groove portion 503b is thinner than a cylinder member 401.

In the present modification, when a treatment instrument 400 is pulled outside, the cylinder member 401 passes the holding portion 503. At this time, the cylinder member 401 shifts from the holding portion 503 into the forceps plug portion 36, because the cylinder member is thicker than the holding portion 503. Consequently, the treatment instrument 400 also shifts into the forceps plug portion 36. Simultaneously, the other part 301b into which the cylinder member 401 is inserted shifts into the forceps plug portion 36. That is, by a pulling operation of the treatment instrument 400, the other part 301b held by the holding portion 503 is released.

Thus, in the present modification, by the pulling operation of the treatment instrument 400, the other part 301b can be removed, and hence, time and labor in the removing can be omitted.

### [Second Embodiment]

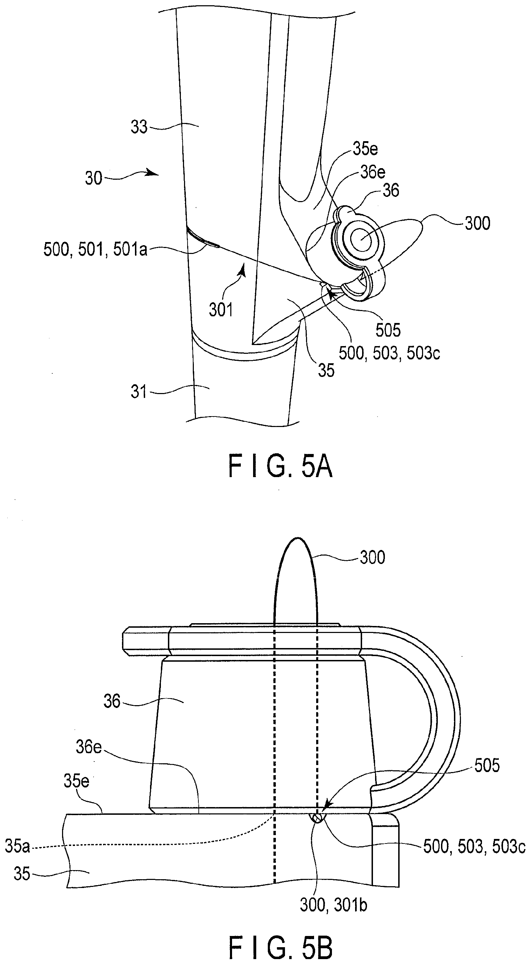

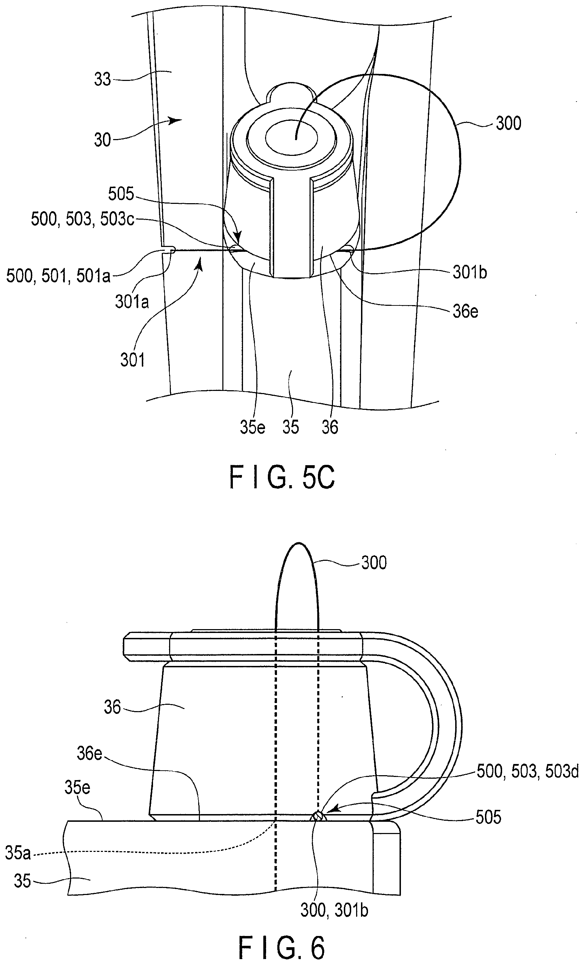

Hereinafter, respects different from those of the first embodiment will only be described with reference to FIG. 5A, FIG. 5B and FIG. 5C.

### [Holding Portion 501]

A holding portion 501 is disposed between a treatment instrument insertion port portion 35a and a proximal end portion of an inserting section 20 in an axial direction of an operating section 30. In detail, the holding portion 501 is disposed between a holding portion 503 and a proximal end portion of a main body portion 31 which will be described later.

The holding portion 501 holds one part 301a so that an axial direction of the one part 301a exposed to the outside of an endoscope 10 is orthogonal to the axial direction of the grasping portion 33 of the operating section 30, and for this purpose, the holding portion 501 is disposed so that an axial direction of the holding portion 501 is along an orthogonal direction orthogonal to an axial direction of a grasping portion 33 of the operating section 30.

### [Holding Portion 503]

A treatment instrument inserting portion 35 has, e.g., a planar first facing portion 35e that faces a forceps plug portion 36.

The forceps plug portion 36 has, e.g., a planar second facing portion 36e that faces the first facing portion 35e.

In a state where the forceps plug portion 36 is inserted to the treatment instrument insertion port portion 35a, the holding portion 503 is disposed in the planar first facing portion 35e that faces the forceps plug portion 36 and that is disposed in the treatment instrument inserting portion 35.

The holding portion 503 has a groove portion 503c with which another part 301b is engageable. The groove portion 503c is notched in an opposite direction to a position at which the forceps plug portion 36 is disposed so that the groove portion 503c is covered with the second facing portion 36e when the forceps plug portion 36 is inserted to the treatment instrument insertion port portion 35a. When the forceps plug portion 36 is inserted to the treatment instrument insertion port portion 35a, the groove portion 503c is covered with the second facing portion 36e, thereby forming a holding hole portion 505 holding the other part 301b.

An axial direction of the groove portion 503c is disposed along, e.g., a planar direction of the first facing portion 35e. The axial direction of the groove portion 503c is disposed along a direction orthogonal to an axial direction of the treatment instrument insertion port portion 35a. That is, the groove portion 503c is disposed to cross the treatment instrument inserting portion 35 and to passed through the treatment instrument inserting portion 35.

An inner diameter of the groove portion 503c is substantially the same as an outer diameter of a guide member 300, and an inner shape of the groove portion 503c is substantially the same as an outer shape of the guide member 300, so that the groove portion 503c engages with the guide member 300. Consequently, the guide member 300 engages with the groove portion 503c, whereby the holding portion 503 holds the other part 301b, and the holding portion 503 fixes a proximal end portion 301 of the guide member 300 to the treatment instrument insertion port portion 35a. The groove portion 503c has, e.g., a U-shape or a concave shape.

### [Operation]

The guide member 300 is inserted to the holding hole portion 505, after the forceps plug portion 36 is inserted to the treatment instrument insertion port portion 35a to form the holding hole portion 505, or the guide member 300 engages with the groove portion 503c, then the forceps plug portion 36 is then inserted to the treatment instrument insertion port portion 35a and the holding hole portion 505 is formed.

Under such situations, the guide member 300 of the present embodiment is disposed so that the guide member reaches from the forceps plug portion 36 toward the holding hole portion 505 via a lateral side of the forceps plug portion 36 and so that the guide member does not interfere with, e.g., the operating section 30, after the guide member is pulled out from the forceps plug portion 36. At this time, the guide member 300 reaches from the forceps plug portion 36 toward the holding hole portion 505 via the lateral side of the forceps plug portion 36 to draw, e.g., a loop. Further, the guide member 300, together with the groove portion 503c, is covered with the forceps plug portion 36. Further, the guide member 300 passes the holding hole portion 505, then reaches from the holding hole portion 505 toward the holding portion 501, and is further held by the holding portion 501.

### [Effect]

In the present embodiment, the holding portion 503 is covered with the forceps plug portion 36, whereby the guide member 300 can be prevented from being abruptly removed. In the present embodiment, simultaneously with an attaching operation of the forceps plug portion 36 to the treatment instrument insertion port portion 35a, the guide member 300 can be attached to the groove portion 503c, and time and labor of the operation can be decreased.

It is to be noted that in the present embodiment, the holding portion 503 is disposed in the first facing portion 35e, but the present invention does not have to be limited to this embodiment. Hereinafter, Modifications 1 and 2 will be described.

### [Modification 1]

As shown in FIG. 6, in a state where a forceps plug portion 36 is inserted to a treatment instrument insertion port portion 35a, a holding portion 503 may be disposed in a planar second facing portion 36e that faces a treatment instrument inserting portion 35 and that is disposed in the forceps plug portion 36.

The holding portion 503 has a groove portion 503d with which another part 301b is engageable. The groove portion 503d is notched in an opposite direction to a position at which the treatment instrument insertion port portion 35a is disposed so that the groove portion 503d is covered with a first facing portion 35e when the forceps plug portion 36 is inserted to the treatment instrument insertion port portion 35a. When the forceps plug portion 36 is inserted to the treatment instrument insertion port portion 35a, the groove portion 503d is covered with the first facing portion 35e, thereby forming the holding hole portion 505 holding the other part 301b.

An axial direction of the groove portion 503d is disposed along, e.g., a planar direction of the second facing portion 36e. The axial direction of the groove portion 503d is disposed along an orthogonal direction to an axial direction of the forceps plug portion 36. In the present modification, the forceps plug portion 36 is attached to the treatment instrument insertion port portion 35a so that a guide member 300 passing the groove portion 503d does not interfere with a grasping portion 33. The groove portion 503d has, e.g., a U-shape or a concave shape.

### [Modification 2]

The present modification is a combination of the second embodiment and Modification 1 of the second embodiment.

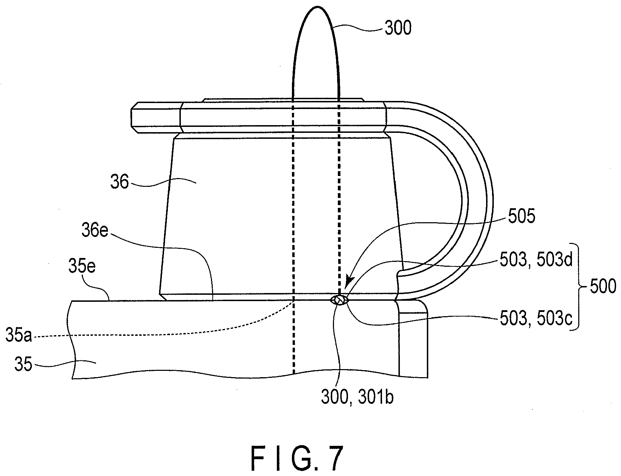

That is, as shown in FIG. 7, in a state where a forceps plug portion 36 is inserted to a treatment instrument insertion port portion 35a, a holding portion 503 is disposed in both a first facing portion 35e and a second facing portion 36e.

The holding portion 503 has groove portions 503c and 503d with which another part 301b is engageable.

When the forceps plug portion 36 is inserted into the treatment instrument insertion port portion 35a, one groove portion 503c disposed in the first facing portion 35e faces the other groove portion 503d disposed in the second facing portion 36e, thereby forming a holding hole portion 505 holding the other part 301b.

### [Conclusion]

In a summary of the second embodiment, Modification 1 and Modification 2, in a state where a forceps plug portion 36 is inserted to a treatment instrument insertion port portion 35a, a holding portion 503 is disposed in at least one of a first facing portion 35e that faces the forceps plug portion 36 and that is disposed in a treatment instrument inserting portion 35 and a second facing portion 36e that faces the treatment instrument inserting portion 35 and that is disposed in the forceps plug portion 36. The holding portion 503 has a groove portion with which the other part 301b is engageable.

The present invention is not limited to the above embodiments as they are, and in an implementation stage, constitutional elements can be modified and embodied without departing from the gist. Additionally, constitutional elements disclosed in the above embodiments can suitably be combined to form various inventions.

## Claims

1. A holding mechanism for an endoscopic guide member which holds the guide member that is inserted to a lumen through an inserting section of an endoscope to be inserted to the lumen and that projects from an opening portion disposed in the inserting section to guide a treatment instrument, the holding mechanism comprising:
a first holding portion which is disposed in an operating section that operates the endoscope and that is coupled to a proximal end portion of the inserting section, the first holding portion holding, in the operating section, a part of the guide member which is exposed from the endoscope on a proximal end side of the guide member in a state where a distal end portion of the guide member projects from the opening portion; and
a second holding portion which is disposed at a position different from that of the first holding portion in the operating section, the second holding portion holding the guide member at a position different from that of the part held by the first holding portion on the proximal end portion side of the guide member and in an orientation different from an orientation of the first holding portion.

2. The holding mechanism according to claim 1,
wherein at least one of the first holding portion and the second holding portion is disposed to hold the guide member in a direction orthogonal to an axial direction of a treatment instrument insertion port portion which the guide member is inserted to the endoscope.

3. The holding mechanism according to claim 1,
wherein the first holding portion is disposed between a treatment instrument insertion port portion disposed in the operating section to insert the treatment instrument and the guide member to the endoscope and the proximal end portion of the inserting section, and
the second holding portion is disposed between the first holding portion and the proximal end portion of the inserting section.

4. The holding mechanism according to claim 3,
wherein at least one of the first holding portion and the second holding portion is disposed so that an axial direction of the one holding portion is along an orthogonal direction orthogonal to an axial direction of the operating section.

5. The holding mechanism according to claim 4,
wherein at least one of the first holding portion and the second holding portion has a groove portion which is formed so that a part of the exterior portion of the operating section is notched and with which a part of the proximal end portion of the guide member is engageable.

6. The holding mechanism according to claim 1,
wherein at least one of the first holding portion and the second holding portion is disposed in the operating section so that the one holding portion is integrated with the exterior portion of the operating section.

7. The holding mechanism according to claim 6,
wherein the second holding portion is disposed in an inner peripheral surface of a forceps plug portion inserted to a treatment instrument insertion port portion disposed in the operating section to insert the treatment instrument and the guide member to the endoscope.

8. The holding mechanism according to claim 7,
wherein the second holding portion has a groove portion which is disposed along a longitudinal axis direction of the forceps plug portion and with which another part of the proximal end portion of the guide member is engageable.

9. The holding mechanism according to claim 1,
wherein in a state where a forceps plug portion is inserted to a treatment instrument insertion port portion disposed in a treatment instrument inserting portion of the operating section to insert the treatment instrument and the guide member to the endoscope,
the second holding portion is disposed in at least one of:
a first facing portion that faces the forceps plug portion and is disposed in the treatment instrument inserting portion; and
a second facing portion that faces the treatment instrument inserting portion and is disposed in the forceps plug portion.

10. The holding mechanism according to claim 1,
wherein at least one of the first holding portion and the second holding portion has a U-shaped projection that projects from an outer surface of the operating section.

11. The holding mechanism according to claim 1,
wherein at least one of the first holding portion and the second holding portion has an elastic body which holds the guide member so that the one holding portion is integrated with the exterior portion of the operating section.

12. An endoscope which comprises the holding mechanism according to claim 1.

13. The endoscope according to claim 12,
which is a side view type endoscope wherein the opening portion is disposed in a side surface of a distal end portion of the inserting section.
